# FASCICULE DE BREVET EUROPEEN

(11) **EP 1 233 809 B1**
(45) Date de publication et mention de la délivrance du brevet: **29.09.2004**
(21) Numéro de dépôt: 00985371.4
(22) Date de dépôt: 30.11.2000
(51) Int. Cl.: A61M 39/10

(54) **RACCORDEMENT NON LUER D'UN CONNECTEUR MALE AVEC UN CONNECTEUR FEMELLE POUR DISPOSITIFS MEDICAUX**
NICHT LUERARTIGER ANSCHLUSS EINES MÄNNLICHEN AN EIN WEIBLICHES VERBINDUNGSSTÜCK FÜR MEDIZINVORRICHTUNGEN
NON-LUER-LOCK CONNECTION OF A MALE CONNECTOR WITH A FEMALE CONNECTOR FOR MEDICAL DEVICES

(30) Priorité: 02.12.1999 FR 9915191
(43) Date de publication de la demande: 28.08.2002
(73) Titulaire: CAIR L.G.L., 69380 Civrieux d'Azergues (FR)
(72) Inventeur: LOPEZ, Georges, Antoine, F-69290 Craponne (FR); GARREAU, Michel, F-69340 Francheville (FR)
(74) Mandataire: Vuillermoz, Bruno
(86) Numéro de dépôt international: PCT/FR2000/003349
(87) Numéro de publication internationale: WO 2001/039827

(56) Documents cités:
- EP-A- 0 397 103
- WO-A-93/20772
- WO-A-97/21951
- GB-A- 2 202 148
- US-A- 4 161 949
- US-A- 5 613 949

## Description

L'invention se rapporte à un connecteur mâle non luer destiné à équiper un premier dispositif médical en vue de son raccordement avec le connecteur femelle correspondant d'un second dispositif médical. Elle concerne donc également un connecteur femelle non luer susceptible de s'adapter sur ledit connecteur mâle. Elle a enfin pour objet les dispositifs médicaux équipés des connecteurs précités.

Dans la suite de la description et dans les revendications, on entend par le terme de dispositif médical :
- les sondes, aussi bien les sondes d'abord entéral (sonde de nutrition entérale, sonde respiratoire, sonde chirurgicale...) que les sondes d'abord génito-urinaire (sonde urinaire, sonde vésicale, sonde chirurgicale...) ou encore rectale (sonde rectale, sonde chirurgicale...) de même que ;
- tout dispositif de prélèvement du contenu d'un conteneur sous forme notamment de tube plongeur ;
- les conteneurs (poche, flacon, seringue orale, seringue de gavage, biberon, etc ...) ;
- les prolongateurs de sonde ;
- les dispositifs de nutrition entérale par gravité ou par pompe ;
- les raccords et adaptateurs (raccord en Y, adaptateur de connexions, etc..) ;
- les bouchons.

Les connecteurs mâle et femelle non luer de l'invention sont plus particulièrement décrits par la suite en relation avec les lignes de nutrition entérale.

Les lignes de nutrition entérale comprennent de un à trois éléments, en fonction de leurs utilisation qui sont :
- un conteneur d'aliments ou de nutriments, ces derniers comprenant non seulement les espèces chimiques utilisables telles quelles ou sans digestion mais également les médicaments ;
- une sonde de nutrition entérale, c'est à dire un tube flexible à un ou plusieurs orifices muni d'un raccord femelle, conçu pour introduire des nutriments ou aliments dans les voies gastro-intestinales ;
- un dispositif de nutrition entérale.

Les dispositifs de nutrition entérale sont des dispositifs médicaux par lesquels des aliments ou nutriments sont transférés d'un conteneur d'aliments ou de nutriments à une sonde de nutrition entérale. En pratique, ces dispositifs se présentent sous forme d'une tubulure flexible, dont l'une des extrémités est équipée d'un moyen d'effectuer un raccordement efficace avec un conteneur de nutriments, tandis que l'autre extrémité est équipée d'un connecteur permettant le raccordement avec une sonde de nutrition entérale. Parmi les dispositifs de nutrition entérale, on distingue les tubulures dites de gravité, les tubulures pour pompes à nutrition entérale et les prolongateurs en particulier lors d'utilisation de pousse seringue.

En pratique, le conteneur, le dispositif de nutrition et la sonde de nutrition peuvent être utilisés de diverses façons.

Ainsi, le conteneur, notamment lorsqu'il se présente sous forme d'une seringue orale, peut être mis au contact direct de la bouche du patient.

Selon une autre utilisation, l'embout mâle du conteneur peut être connecté à l'embout femelle de la sonde de nutrition entérale, cette dernière étant introduite chez le patient par le nez ou la bouche ou par gastrostomie, jéjunostomie ou oesophagostomie.

La troisième utilisation nécessite la mise en place entre le conteneur et la sonde, d'un dispositif de nutrition entérale muni aux deux extrémités respectivement d'un embout mâle destiné à coopérer avec l'embout femelle de la sonde ou d'un embout femelle destiné à coopérer avec l'embout mâle du conteneur.

L'ensemble de ces mises en oeuvre est parfaitement connu de l'homme du métier et ne sera donc pas davantage explicité.

La norme EN 1615 a établi un certain nombre de prescriptions pour les sondes et dispositifs de nutrition entérale, qui imposent notamment que le raccord du dispositif de nutrition entérale ne doit pas pouvoir être connecté à une voie d'abord munie d'un assemblage conique femelle (luer) ou d'un assemblage à verrouillage femelle (luer-lock).

L'expression "luer" désigne tout assemblage conique femelle ou mâle à 6 % précisément décrit dans la norme EN 1707. De tels assemblages coniques équipent tous les dispositifs médicaux utilisables en voies d'abord parentérale (veine ou artère) notamment pour la perfusion, la transfusion etc...Dans tous ces cas, la voie d'abord est luer femelle (aiguille, cathéter court, cathéter de voie centrale) pour permettre la connexion d'une seringue (luer mâle) ou d'un perfuseur (luer mâle côté patient).

A l'inverse, l'expression "non luer" désigne tout assemblage ne répondant pas aux prescriptions de la norme précitée.

La norme EN 1615 impose également que toutes les parties, les raccordements et les joints du dispositif de nutrition entérale, ainsi que ceux de la sonde de nutrition entérale doivent supporter une force de traction linéaire de 15 Newtons, sans qu'il se produise de déconnexion, de rupture ou de déchirure.

Enfin, la norme précitée exige que toutes les parties, les raccordements et les joints du dispositif de nutrition entérale et de la sonde soient aptes à supporter la pression maximale d'utilisation sans fuite de liquide.

Par extension, cette norme concerne également les conteneurs lorsqu'ils sont connectés directement sur la sonde de nutrition entérale.

Dès lors, le premier problème que se propose de résoudre l'invention est de fournir un connecteur mâle destiné à équiper un dispositif médical et en particulier un dispositif de nutrition entérale ou un conteneur en vue de leur raccordement à une sonde, notamment de nutrition entérale qui soit conforme aux exigences de la norme EN 1615.

Pour ce faire, l'invention propose tout d'abord un connecteur mâle non luer destiné à équiper un premier dispositif médical en vue de son raccordement au connecteur femelle correspondant d'un second dispositif médical, comprenant un corps pourvu d'une lumière traversante formant canal.

Ce connecteur se caractérise en ce que l'une des deux extrémités dudit corps est munie d'un bourrelet ménagé sur toute ou partie de sa périphérie, ledit corps muni du bourrelet étant destiné à coopérer avec le connecteur femelle correspondant dudit second dispositif médical et permettre une déconnexion volontaire pour une force de traction linéaire supérieure à 15 Newtons.

En d'autres termes, le connecteur mâle objet de l'invention est inapte à s'adapter à des connecteurs femelle luer ou femelle à verrouillage luer, interdisant donc tout raccordement à un système intravasculaire parentéral ou tout autre dispositif médical à raccord femelle luer. De plus, l'agencement spécifique d'un bourrelet à l'extrémité du corps permet d'interdire toute déconnexion avec le connecteur femelle sous une force de traction linéaire inférieure à 15 Newtons, la déconnexion volontaire pour des forces supérieures étant donc possible.

Dans une première forme de réalisation, le corps se présente sous forme d'un cylindre, ledit bourrelet étant ménagé à l'extrémité du cylindre.

Dans une seconde forme de réalisation, le corps se présente sous forme d'un cône, ledit bourrelet étant ménagé à l'extrémité de la pointe du cône.

Avantageusement, le bourrelet est ménagé sur toute la périphérie de l'extrémité du corps.

Dans la suite de la description et dans les revendications, par le terme "bourrelet" on désigne une protubérance présentant dans au moins une de ses régions une forme arrondie apte à interdire la déconnexion du connecteur mâle avec le connecteur femelle correspondant pour une force de traction inférieure à 15 Newtons. Le bourrelet peut ainsi adopter diverses formes telles qu'une forme cylindrique, elliptique ou encore quadrilatérale dont les angles sont arrondis et ce, de façon non limitative.

Dans le cas d'une forme cylindrique, le bourrelet présente sur sa face frontale un rayon de courbure compris entre 0,5 et 1,5 mm, avantageusement 1 mm. Dans une forme de réalisation préférée, et afin de faciliter la déconnexion, le bourrelet présente, en direction de l'arrière du connecteur, un rayon de courbure compris entre 0,1 et 0,6 mm. Selon une autre caractéristique, le bourrelet présente une épaisseur comprise entre 1 et 2 mm, avantageusement 1,5mm.

Selon une autre caractéristique, le connecteur mâle présente des moyens d'obturation escamotables.

Dans une autre forme de réalisation, le connecteur femelle décrit par la suite, fait fonction de moyens d'obturation.

Pour permettre son adaptation à un premier dispositif médical, le corps se prolonge à son extrémité opposée à celle munie du bourrelet par des moyens de coopération.

Ainsi, lorsque le dispositif médical est un conteneur se présentant sous forme d'une poche ou d'un flacon muni d'un embout de connexion sous forme d'un pas de vis, les moyens de coopération se présentent sous forme d'un percuteur vissable destiné à coopérer avec le pas de vis correspondant de l'embout de connexion dudit conteneur.

Lorsque le conteneur se présente sous forme d'une seringue de gavage ou d'une seringue orale, c'est à dire d'une seringue dont l'embout est un embout mâle cylindrique ou conique, les moyens de coopération se présentent sous forme d'un cylindre ou d'un cône femelle pourvu d'une lumière traversante destiné à coopérer avec le cylindre ou le cône mâle correspondant de la seringue.

Dans une autre forme de réalisation, le connecteur mâle non luer de l'invention est agencé directement en lieu et place de l'embout de connexion du conteneur, que ce soit une poche, un flacon ou une seringue de gavage ou orale.

Dès lors, l'invention concerne également un conteneur, dont le connecteur mâle non luer est destiné à coopérer avec un connecteur femelle correspondant caractérisé en ce qu'il se présente sous forme d'un corps, dont l'extrémité libre est munie d'un bourrelet ménagé sur toute ou partie de sa périphérie, ledit corps muni d'un bourrelet étant destiné à coopérer avec un connecteur femelle correspondant.

Comme déjà évoqué, le connecteur mâle de l'invention doit également pouvoir s'adapter sur le tuyau flexible constituant les dispositifs médicaux du type dispositifs de nutrition entérale.

Pour ce faire, les moyens de coopération se présentent sous forme d'un embout de forme générale tubulaire pourvu d'une lumière traversante destinée à recevoir le tuyau flexible, la section de la lumière traversante étant sensiblement égale à celle du tuyau flexible.

Le second problème que se propose de résoudre l'invention est de fournir un connecteur femelle qui puisse s'adapter sur le connecteur mâle ci-avant décrit, qui ne puisse pas s'adapter sur un connecteur mâle luer, le connecteur femelle étant susceptible de coopérer avec le tuyau flexible d'une sonde, (y compris comme déjà dit les dispositifs de prélèvement du type tube plongeur), ou l'extrémité du tuyau flexible d'un dispositif de nutrition entérale, destinée à coopérer avec le conteneur.

Dès lors, l'invention a également pour objet un connecteur femelle non luer sous forme d'un élément tubulaire pourvu d'une lumière traversante formant canal, dont l'une des extrémités est destinée à coopérer avec un dispositif médical, caractérisée en ce que le canal au voisinage de l'extrémité opposée, d'une part, présente une section correspondant sensiblement à celle du connecteur mâle précédemment décrit et d'autre part, est pourvu d'une gorge annulaire destinée à coopérer avec le bourrelet ménagé à l'extrémité dudit connecteur mâle.

Pour permettre la coopération du bourrelet avec la gorge, ladite gorge présente une forme complémentaire à celle du bourrelet en pratique, cylindrique, elliptique ou encore quadrilatérale munie d'angles arrondis.

Dans une forme de réalisation avantageuse, la partie arrière de la gorge destinée à coopérer avec la partie arrière arrondie à enlever du bourrelet présente un angle vif permettant d'interdire toute déconnexion sous une force de traction linéaire inférieure à 15 Newtons.

Lorsque le dispositif médical se présente sous forme d'une sonde ou d'un dispositif de nutrition entérale, la section du canal au voisinage de l'extrémité destinée à coopérer avec le tuyau flexible dudit dispositif correspond sensiblement à celle du tuyau flexible.

Dans une forme de réalisation avantageuse, le connecteur femelle est équipé d'un bouchon destiné à obturer l'extrémité apte à recevoir ledit connecteur mâle.

Cependant, dans une autre forme de réalisation, le connecteur mâle précédemment décrit est utilisé pour boucher le connecteur.

L'invention se rapporte également à une sonde constituée d'un tuyau flexible, dont l'une des extrémités coopère avec l'extrémité correspondante du connecteur femelle précédemment décrit.

Comme déjà dit, un dispositif de nutrition entérale sépare dans certaines formes d'utilisations le conteneur de la sonde. Dès lors, il est nécessaire que ce dispositif soit muni d'agencements permettant non seulement son raccord au conteneur, mais également à la sonde.

Autrement dit, l'invention a pour objet un dispositif de nutrition entérale comprenant une tubulure flexible, dont les deux extrémités sont munies respectivement du connecteur mâle et du connecteur femelle caractéristiques de l'invention, le connecteur mâle étant destiné à coopérer avec le connecteur femelle correspondant de la sonde et le connecteur femelle étant destiné à coopérer avec le connecteur mâle correspondant du conteneur.

Comme déjà dit, figurent également parmi les dispositifs médicaux, tout raccord et notamment les raccords en Y. Ce type de raccord est susceptible de s'adapter notamment sur les tubulures flexibles en particulier pour permettre la jonction avec un autre dispositif médical.

Dès lors, l'invention concerne aussi un raccord en Y, dont au moins une des extrémités est équipée du connecteur mâle de l'invention ou du connecteur femelle de l'invention.

Les adaptateurs font également partie des dispositifs médicaux concernés par l'invention. Les adaptateurs permettent en particulier le branchement de lignes de nutrition par gravité ou pompe disposant d'un cône non luer. En conséquence, l'invention concerne également un adaptateur, dont une des extrémités est équipée du connecteur mâle de l'invention, l'autre extrémité étant apte à recevoir en particulier mais de façon non limitative une ligne de nutrition.

L'invention et les avantages qui en découlent ressortiront mieux des exemples de réalisation suivants à l'appui des figures annexées.

La figure 1 est une représentation d'une ligne de nutrition entérale comprenant un conteneur d'aliments ou de nutriments, un dispositif de nutrition entérale et une sonde de nutrition entérale équipés des différents connecteurs caractéristiques de l'invention.
La figure 2 est une représentation d'une seringue orale caractéristique de l'invention.
La figure 3 est une représentation du connecteur femelle destiné à coopérer avec le connecteur mâle caractéristique de l'invention.
La figure 4 est une représentation du connecteur mâle destiné à équiper l'extrémité d'un prolongateur de sonde.

Dans cet exemple, l'invention est plus particulièrement décrite dans son application à la nutrition entérale pédiatrique ou en néonatologie.

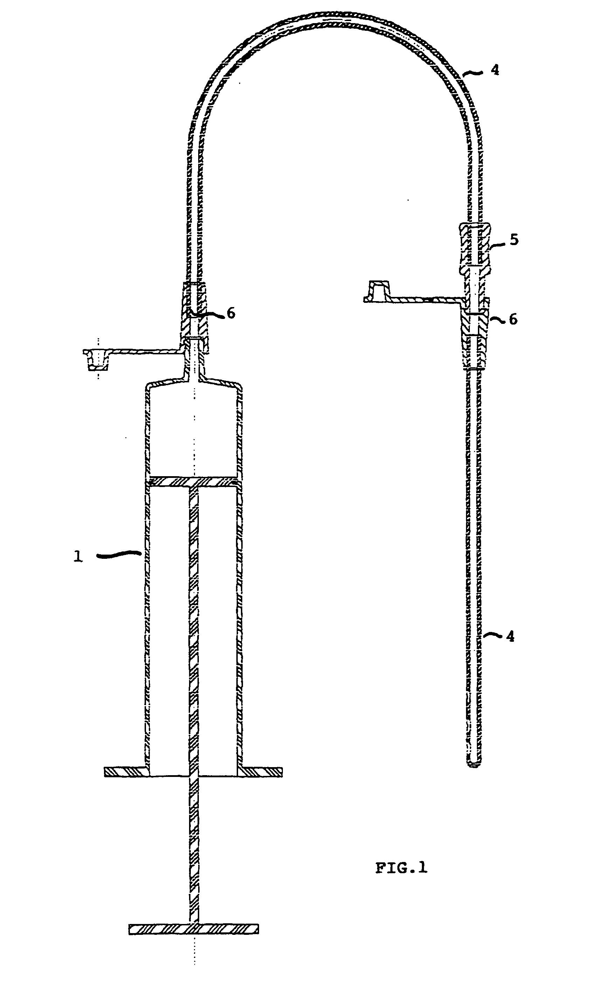

Sur la figure 1, on a représenté une ligne de nutrition entérale comprenant un conteneur (1) sous forme d'une seringue orale, un dispositif de nutrition (2) et une sonde de nutrition entérale (3).

La sonde et/ou le dispositif de nutrition se présentent sous forme d'un tuyau flexible (4) équipé des connecteurs mâle (5) et femelle (6) de l'invention.

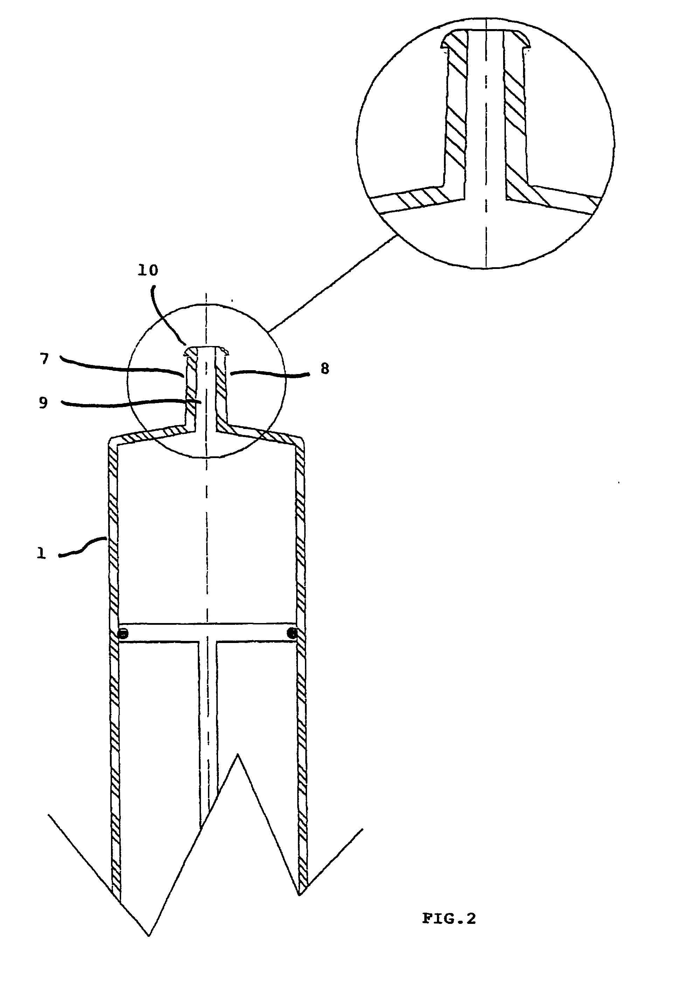

Sur la figure 2, on a représenté le corps de seringue orale (1), dont l'embout de connexion (7) se présente sous forme d'un cône (8) pourvu d'une lumière traversante (9), dont l'extrémité de la pointe est munie d'un bourrelet (10) ménagé sur toute sa circonférence. Comme le montre cette même figure, le bourrelet présente sur sa face avant un premier rayon de courbure (10a), en pratique de l'ordre de 1 mm et sur sa partie arrière un second rayon de courbure (10b) de l'ordre de 0,5 mm destiné à faciliter la déconnexion de la gorge correspondante d'un connecteur femelle.

Comme déjà dit, une telle seringue peut être mise au contact de la bouche du patient ou connectée soit directement, soit par le biais d'un dispositif de nutrition entérale, à une sonde de nutrition.

Dans ce cas, il est nécessaire que la sonde ou le dispositif de nutrition soient équipés d'un connecteur femelle qui puisse coopérer avec le connecteur mâle de la seringue.

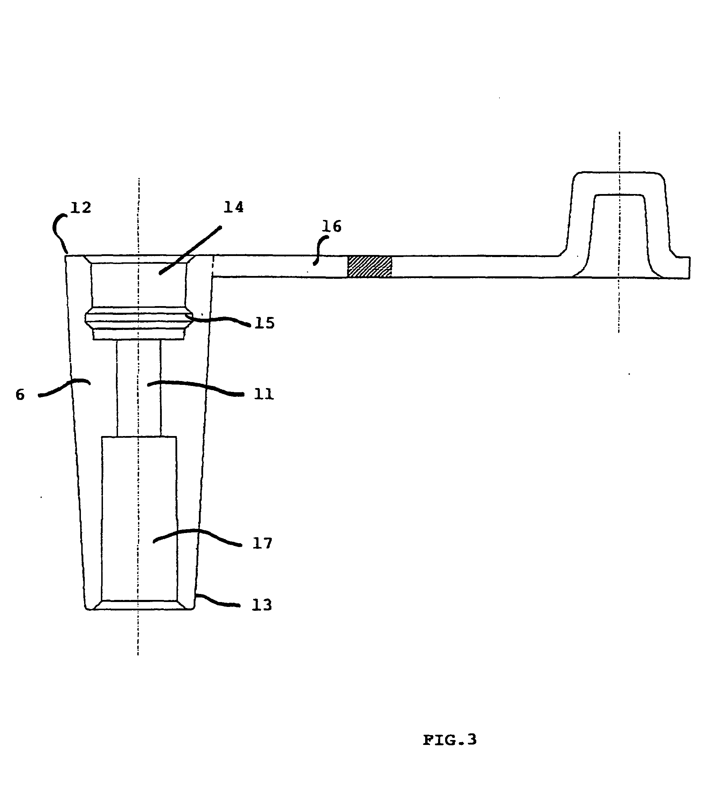

Sur la figure 3, on a représenté un tel connecteur femelle (6) de forme générale tubulaire, pourvu d'une lumière traversante (11) formant canal, dont l'une des extrémités (12) est destinée à coopérer avec l'embout mâle (7) précédemment décrit, tandis que l'extrémité (13) est destinée à recevoir la tubulure flexible (4) d'une sonde, d'un prolongateur de sonde ou d'un dispositif de nutrition.

Comme le montre cette figure, la section du canal (11), au voisinage (14) de l'extrémité (12) du connecteur femelle est de forme conique sensiblement identique à celle (8) de l'embout de connexion (7) et pourvue d'une gorge annulaire (15) destinée à coopérer avec le bourrelet (10) caractéristique du connecteur mâle de la seringue (1). Plus précisément, la gorge annulaire (15) présente sur sa face avant (15a) un rayon de courbure sensiblement supérieur à celui correspondant du bourrelet (10a) tandis que la face arrière (15b) présente un angle vif destiné à empêcher toute déconnexion du connecteur mâle sous une force de traction linéaire inférieure à au moins 15 Newtons.

Plus précisément, la section et la profondeur de la partie (14) du canal (11), correspond sensiblement à la section et la longueur de la partie du connecteur mâle destinée à pénétrer dans le connecteur (7).

De la sorte, le connecteur femelle se trouve être non luer de par sa profondeur et son diamètre interne, de sorte qu'il ne peut pas être connecté avec un connecteur mâle luer.

Dès lors, la connexion se fait par encliquetage du cône (8) et du bourrelet (10) de la seringue dans le cône (14) et la gorge annulaire (15) correspondant du connecteur femelle. Une telle connexion permet de répondre aux exigences de la norme EN 1615 déjà citée, dans la mesure où un tel dispositif supporte une force de traction linéaire d'au moins 15 Newtons sans qu'il ne se produise de déconnexion.

Pour permettre la fermeture de la sonde après déconnexion du conteneur d'aliments ou du dispositif de nutrition, le connecteur femelle est équipé d'un capuchon (16).

Comme déjà dit, l'extrémité opposée (13) du connecteur femelle est destinée à recevoir le tuyau flexible (4) d'un prolongateur de sonde ou d'une sonde. Dès lors, sa section (17) est sensiblement égale à la section du tuyau flexible (4), la solidarisation du connecteur et de la tubulure étant réalisée par tout moyen connu.

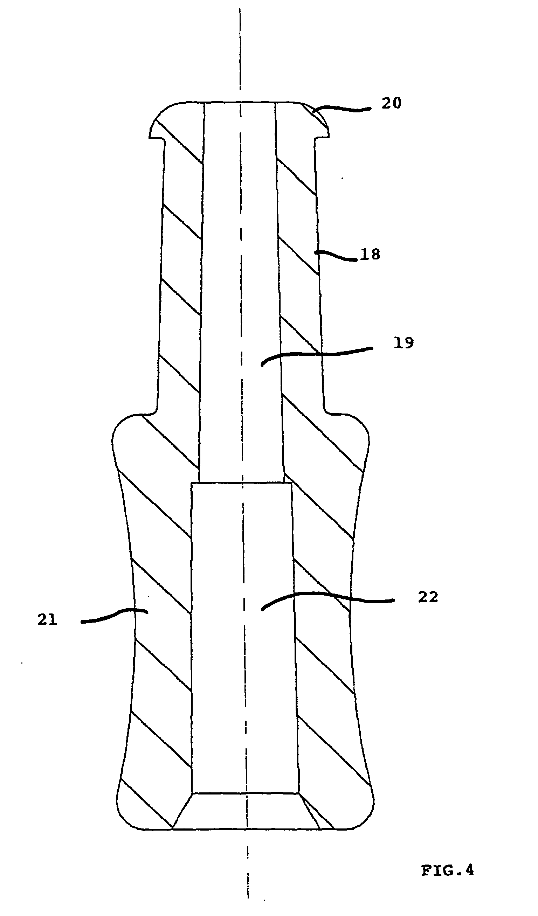

Sur la figure 4, on a représenté le connecteur mâle (5) caractéristique de l'invention lequel est destiné à coopérer avec l'extrémité de la tubulure flexible (4) du dispositif de nutrition entérale, opposée à celle munie du connecteur femelle (6).

Comme le montre cette figure, ce connecteur (5) se présente sous forme d'un cône (18) muni d'une lumière traversante (19) et dont la pointe est pourvue d'un bourrelet (20) ménagé sur toute sa circonférence. De même que précédemment, le bourrelet présente sur sa face avant un premier rayon de courbure (20a), en pratique de l'ordre de 1 mm, et, sur sa partie arrière un second rayon de courbure (20b) en pratique de l'ordre de 0,5 mm. Le cône (18) se prolonge par un élément tubulaire (21) muni d'une lumière traversante (22), la section de la lumière traversante (22) étant sensiblement égale à celle de la tubulure flexible (4) du dispositif de nutrition entérale.

Les avantages de l'invention ressortent bien de la description qui précède. On notera notamment la complétude des connecteurs mâle et femelle de l'invention avec en particulier la norme EN 1615. De tels connecteurs peuvent en outre équiper toute sonde ou prolongateur de sonde, dispositif de nutrition entérale, conteneur du type seringue orale, seringue de gavage, poche, flacon, etc...

## Revendications

1. Connecteur mâle (5) non luer destiné'à équiper un premier dispositif médical en vue de son raccordement au connecteur femelle (6) correspondant d'un second dispositif médical, comprenant un corps (18) pourvu d'une lumière traversante (19) formant canal, **caractérisé en ce que** l'une des deux extrémités dudit corps est muni d'un bourrelet (20) ménagé sur toute ou partie de sa périphérie, ledit corps muni d'un bourrelet étant destiné à coopérer avec le connecteur femelle correspondant dudit second dispositif médical et permettre une déconnexion volontaire pour une force de traction linéaire supérieure à 15 Newtons.

2. Connecteur mâle selon la revendication 1, **caractérisé en ce que** le corps (18) se présente sous forme d'un cylindre, ledit bourrelet (20) étant ménagé à l'extrémité du cylindre.

3. Connecteur mâle selon la revendication 1, **caractérisé en ce que** le corps (18) se présente sous forme d'un cône, ledit bourrelet (20) étant ménagé à l'extrémité de la pointe du cône.

4. Connecteur mâle selon la revendication 1, **caractérisé en ce que** le bourrelet (20)est ménagé sur toute la périphérie de l'extrémité du corps (18).

5. Connecteur mâle selon la revendication 1, **caractérisé en ce que** le corps prolonge à l'extrémité opposée à celle munie du bourrelet par des moyens de coopération avec ledit premier dispositif médical.

6. Connecteur mâle selon la revendication 5, **caractérisé en ce que**, lorsque dispositif médical est un conteneur du type poche ou flacon muni d'un embout de onnexion sous forme d'un pas de vis, les moyens de coopération se présentent ous forme d'un percuteur vissable destiné à coopérer avec le pas de vis orrespondant de l'embout de connexion dudit conteneur.

7. Connecteur mâle selon la revendication 5, **caractérisé en ce que**, lorsque le dispositif médical est un conteneur du type seringue de gavage ou orale, dont l'embout de connexion est un cylindre ou cône mâle, les moyens de coopération se présentent sous forme d'un cylindre ou cône femelle pourvu d'une lumière traversante destiné à coopérer avec le cylindre ou cône mâle de ladite seringue.

8. Connecteur mâle selon la revendication 5, **caractérisé en ce que**, lorsque le dispositif médical se présente sous la forme d'un dispositif de nutrition entérale muni d'un tube flexible, les moyens de coopération se présentent sous forme d'un embout de forme générale tubulaire (21) pourvu d'une lumière traversante (22) destinée à recevoir le tube flexible (4), la section de la lumière traversante étant sensiblement égale à celle dudit tube flexible.

9. Conteneur intégrant le connecteur mâle objet de l'une des revendications 1 à 8.

10. Conteneur selon la revendication 9, **caractérisé en ce qu'**il s'agit d'une poche ou d'un flacon.

11. Conteneur selon la revendication 9, **caractérisé en ce qu'**il s'agit d'une seringue de gavage ou d'une seringue orale.

12. Connecteur femelle non luer se présentant sous forme d'un élément tubulaire (6) pourvu d'une lumière traversante formant canal (11), dont l'une des extrémités (13) est destinée à coopérer avec un dispositif médical, **caractérisée en ce que** le canal au voisinage de l'extrémité opposée (12) :
• d'une part, présente une section (14) correspondant sensiblement à celle du connecteur mâle, objet de l'une des revendications 1 à 8 ;
• et d'autre part, est pourvu d'une gorge annulaire (15) destinée à coopérer avec le bourrelet ménagé à l'extrémité dudit connecteur mâle.

13. Connecteur femelle selon la revendication 12, **caractérisé en ce que** lorsque le dispositif médical se présente sous forme d'une sonde ou d'un dispositif de nutrition entérale, la section du canal (11) au voisinage de l'extrémité (13) destinée à coopérer avec le tuyau flexible dudit dispositif correspond sensiblement à celle du tuyau flexible.

14. Connecteur femelle selon la revendication 12, **caractérisé en ce qu'**il est équipé d'un bouchon (16) destiné à obturer l'extrémité apte à recevoir le connecteur mâle ou l'embout de la seringue.

15. Sonde composée d'un tuyau flexible, dont une des extrémités est équipée du connecteur femelle objet de l'une des revendications 12 à 14.

16. Dispositif de nutrition entérale comprenant une tubulure flexible, **caractérisé en ce que** les deux extrémités de ladite tubulure sont munies respectivement du connecteur mâle, objet de l'une des revendications 1 à 8 et du connecteur femelle objet de l'une des revendications 12 à 14, le connecteur mâle étant destiné à coopérer avec le connecteur femelle correspondant d'une sonde et le connecteur femelle étant destiné à coopérer avec le connecteur mâle correspondant d'un conteneur.

17. Raccord en Y, dont au moins une des extrémités est équipée du connecteur mâle objet de l'une des revendications 1 à 8 ou du connecteur femelle objet de l'une des revendications 12 à 14.

18. Adaptateur dont une des extrémités est équipée du connecteur mâle objet de l'une des revendications 1 à 8.

## Claims

1. Non-luer-lock male connector (5) designed to equip a first medical device for its connection to the corresponding female connector (6) of a second medical device, comprising a body (18) provided with a through-lumen (19) forming a passage, **characterized in that** one of the two ends of said body is provided with a flange (20) arranged on all or part of its periphery, said body provided with a flange being designed to cooperate with the corresponding female connector of said second medical device and permit deliberate disconnection at a linear tensile force greater than 15 newtons.

2. Male connector according to Claim 1, **characterized in that** the body (18) is in the form of a cylinder, said flange (20) being arranged at the end of the cylinder.

3. Male connector according to Claim 1, **characterized in that** the body (18) is in the form of a cone, said flange (20) being arranged at the end of the point of the cone.

4. Male connector according to Claim 1, **characterized in that** the flange (20) is arranged on the entire periphery of the end of the body (18).

5. Male connector according to Claim 1, **characterized in that** the body, at the end remote from that provided with the flange, is continued via means for cooperation with said first medical device.

6. Male connector according to Claim 5, **characterized in that**, when the medical device is a container of the bag or vial type provided with a connection piece in the form of a screw thread, the means for cooperation are in the form of a screw-on spike designed to cooperate with the corresponding screw thread of the connection piece of said container.

7. Male connector according to Claim 5, **characterized in that**, when the medical device is a container of the gavage syringe or oral syringe type, whose connection piece is a male cone or cylinder, the means for cooperation are in the form of a female cone or cylinder provided with a through-lumen designed to cooperate with the male cone or cylinder of said syringe.

8. Male connector according to Claim 5, **characterized in that**, when the medical device is in the form of an enteral nutrition device provided with a flexible tube, the means for cooperation are in the form of a connection piece of general tubular shape (21) provided with a through-lumen (22) designed to receive the flexible tube (4), the cross section of the through-lumen being substantially equal to that of said flexible tube.

9. Container incorporating the male connector which is the subject of one of Claims 1 to 8.

10. Container according to Claim 9, **characterized in that** it is a bag or a vial.

11. Container according to Claim 9, **characterized in that** it is a gavage syringe or an oral syringe.

12. Non-luer-lock female connector in the form of a tubular element (6) provided with a through-lumen forming a passage (11), one of whose ends (13) is designed to cooperate with a medical device, **characterized in that** the passage near the opposite end (12):
- on the one hand has a cross section (14) corresponding substantially to that of the male connector, which is the subject of one of Claims 1 to 8;
- and, on the other hand, is provided with an annular groove (15) designed to cooperate with the flange arranged at the end of said male connector.

13. Female connector according to Claim 12, **characterized in that**, when the medical device is in the form of an enteral nutrition device or probe, the cross section of the passage (11) near the end (13) designed to cooperate with the flexible tube of said device corresponds substantially to that of the flexible tube.

14. Female connector according to Claim 12, **characterized in that** it is equipped with a stopper (16) designed to close the end receiving the male connector or the connection piece of the syringe.

15. Probe composed of a flexible tube, one of whose ends is equipped with the female connector which is the subject of one of Claims 12 to 14.

16. Device for enteral nutrition comprising a flexible tube, **characterized in that** the two ends of said tube are provided, respectively, with the male connector, which is the subject of one of Claims 1 to 8, and with the female connector, which is the subject of Claims 12 to 14, the male connector being designed to cooperate with the corresponding female connector of a probe, and the female connector being designed to cooperate with the corresponding male connector of a container.

17. Y-shaped connector, at least one of whose ends is equipped with the male connector which is the subject of one of Claims 1 to 8 or the female connector which is the subject of one of Claims 12 to 14.

18. Adapter, one of whose ends is equipped with the male connector which is the subject of one of Claims 1 to 8.

## Patentansprüche

1. Nicht luerartiges männliches Verbindungsteil (5), welches dazu bestimmt ist, eine erste medizinische Vorrichtung auszustatten, um es an ein entsprechendes weibliches Verbindungsteil (6) einer zweiten medizinischen Vorrichtung anzuschließen, und welches einen Körper (18) umfaßt, der mit einer einen Kanal bildenden durchgehenden Öffnung (19) versehen ist, **dadurch gekennzeichnet, daß** eines der beiden Enden des genannten Körpers mit einer Wulst (20) ausgestattet ist, die an der Gesamtheit oder einem Teil seines Umfangs angebracht ist, wobei der genannte mit einer Wulst ausgestattete Körper dazu bestimmt ist, mit dem entsprechenden weiblichen Verbindungsteil der genannten zweiten medizinischen Vorrichtung zusammenzuwirken und eine selbsttätige Trennung bei einer linearen Zugkraft über 15 Newton zu ermöglichen.

2. Männliches Verbindungsteil nach Anspruch 1, **dadurch gekennzeichnet, daß** der Körper (18) die Form eines Zylinders aufweist, wobei die genannte Wulst (20) am Ende des Zylinders angebracht ist.

3. Männliches Verbindungsteil nach Anspruch 1, **dadurch gekennzeichnet, daß** der Körper (18) die Form eines Kegels aufweist, wobei die genannte Wulst (20) am Ende der Spitze des Kegels angebracht ist.

4. Männliches Verbindungsteil nach Anspruch 1, **dadurch gekennzeichnet, daß** die Wulst (20) am gesamten Umfang des Endes des Körpers (18) angebracht ist.

5. Männliches Verbindungsteil nach Anspruch 1, **dadurch gekennzeichnet, daß** der Körper an dem Ende, das dem mit der Wulst ausgestatteten gegenüberliegt, durch Mittel zum Zusammenwirken mit der genannten ersten medizinischen Vorrichtung verlängert ist.

6. Männliches Verbindungsteil nach Anspruch 5, **dadurch gekennzeichnet, daß**, wenn die medizinische Vorrichtung ein Behälter des Typs Beutel oder Fläschchen ist, welcher mit einem Anschlußstück in Form eines Gewindegangs ausgestattet ist, die Mittel zum Zusammenwirken die Form eines aufschraubbaren Einstechteils aufweisen, welches dazu bestimmt ist, mit dem entsprechenden Gewindegang des Anschlußstücks des genannten Behälters zusammenzuwirken.

7. Männliches Verbindungsteil nach Anspruch 5, **dadurch gekennzeichnet, daß**, wenn die medizinische Vorrichtung ein Behälter des Typs Fütter-Spritze oder orale Spritze ist, deren Anschlußstück ein männlicher Zylinder oder Kegel ist, die Mittel zum Zusammenwirken die Form eines weiblichen Zylinders oder Kegels aufweisen, welcher mit einer durchgehenden Öffnung versehen ist, die dazu bestimmt ist, mit dem männlichen Zylinder oder Kegel der genannten Spritze zusammenzuwirken.

8. Männliches Verbindungsteil nach Anspruch 5, **dadurch gekennzeichnet, daß**, wenn die medizinische Vorrichtung die Form einer Vorrichtung zur enteralen Ernährung aufweist, welche mit einem flexiblen Schlauch ausgestattet ist, die Mittel zum Zusammenwirken die Form eines im wesentlichen röhrenförmigen Anschlußstückes (21) aufweisen, welches mit einer durchgehenden Öffnung (22) versehen ist, die dazu bestimmt ist, den flexiblen Schlauch (4) aufzunehmen, wobei der Querschnitt der durchgehenden Öffnung im wesentlichen gleich dem des genannten flexiblen Schlauches ist.

9. Behälter, welcher ein männliches Verbindungsteil umfaßt, welches Gegenstand eines der Ansprüche 1 bis 8 ist.

10. Behälter nach Anspruch 9, **dadurch gekennzeichnet, daß** es sich um einen Beutel oder um ein Fläschchen handelt.

11. Behälter nach Anspruch 9, **dadurch gekennzeichnet, daß** es sich um eine Fütter-Spritze oder um eine orale Spritze handelt.

12. Nicht luerartiges weibliches Verbindungsteil, welches die Form eines röhrenförmigen Elementes (6) aufweist, welches mit einer einen Kanal (11) bildenden durchgehenden Öffnung versehen ist, von deren Enden eines (13) dazu bestimmt ist, mit einer medizinischen Vorrichtung zusammenzuwirken, **dadurch gekennzeichnet, daß** der Kanal nahe dem gegenüberliegenden Ende (12):
• einerseits einen Querschnitt (14) aufweist, welcher im wesentlichen dem des männlichen Verbindungsteils entspricht, welches Gegenstand eines der Ansprüche 1 bis 8 ist;
• und andererseits mit einer Ringnut (15) versehen ist, die dazu bestimmt ist, mit der am Ende des genannten männlichen Verbindungsteils angebrachten Wulst zusammenzuwirken.

13. Weibliches Verbindungsteil nach Anspruch 12, **dadurch gekennzeichnet, daß**, wenn die medizinische Vorrichtung die Form einer Sonde oder einer Vorrichtung zur enteralen Ernährung aufweist, der Querschnitt des Kanals (11) nahe dem Ende (13), welcher zum Zusammenwirken mit dem flexiblen Schlauch der genannten Vorrichtung bestimmt ist, im wesentlichen dem des flexiblen Schlauches entspricht.

14. Weibliches Verbindungsteil nach Anspruch 12, **dadurch gekennzeichnet, daß** es mit einem Stopfen (16) ausgestattet ist, welcher dazu bestimmt ist, das zur Aufnahme des männlichen Verbindungsteils geeignete Ende oder das Anschlußstück der Spritze zu verschließen.

15. Sonde, bestehend aus einem flexiblen Schlauch, von dessen Enden eines mit dem weiblichen Verbindungsteil ausgestattet ist, welches Gegenstand eines der Ansprüche 12 bis 14 ist.

16. Vorrichtung zur enteralen Ernährung, umfassend einen flexiblen Schlauchansatz, **dadurch gekennzeichnet, daß** die beiden Enden des genannten Schlauchansatzes mit dem männlichen Verbindungsteil, welches Gegenstand eines der Ansprüche 1 bis 8 ist, bzw. mit dem weiblichen Verbindungsteil, welches Gegenstand eines der Ansprüche 12 bis 14 ist, ausgestattet ist, wobei das männliche Verbindungsteil dazu bestimmt ist, mit dem entsprechenden weiblichen Verbindungsteil einer Sonde zusammenzuwirken, und wobei das weibliche Verbindungsteil dazu bestimmt ist, mit dem entsprechenden männlichen Verbindungsteil eines Behälters zusammenzuwirken.

17. Y-förmiges Verbindungsteil, von dessen Enden mindestens eines mit dem männlichen Verbindungsteil, welches Gegenstand eines der Ansprüche 1 bis 8 ist, oder mit dem weiblichen Verbindungsteil, welches Gegenstand eines der Ansprüche 12 bis 14 ist, ausgestattet ist.

18. Adapter, von dessen Enden eines mit dem männlichen Verbindungsteil ausgestattet ist, welches Gegenstand eines der Ansprüche 1 bis 8 ist.
